(19) Europäisches Patentamt
European Patent Office
Office européen des brevets

(11) EP 1 611 126 B1

(12) **EUROPÄISCHE PATENTSCHRIFT**

(45) Veröffentlichungstag und Bekanntmachung des Hinweises auf die Patenterteilung:
**28.10.2009 Patentblatt 2009/44**

(21) Anmeldenummer: **04718300.9**

(22) Anmeldetag: **08.03.2004**

(51) Int Cl.:
***C07D 405/12*** *(2006.01)* ***A61K 31/495*** *(2006.01)*

(86) Internationale Anmeldenummer:
**PCT/EP2004/002351**

(87) Internationale Veröffentlichungsnummer:
**WO 2004/087692 (14.10.2004 Gazette 2004/42)**

(54) **CHROMENONINDOLE**

CHROMENONE INDOLES

CHROMENONINDOLE

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**LT LV**

(30) Priorität: **04.04.2003 DE 10315285**

(43) Veröffentlichungstag der Anmeldung:
**04.01.2006 Patentblatt 2006/01**

(73) Patentinhaber: **Merck Patent GmbH**
**64293 Darmstadt (DE)**

(72) Erfinder:
- **SCHIEMANN, Kai**
  **64342 Seeheim-Jugenheim (DE)**
- **BÖTTCHER, Henning**
  **64287 Darmstadt (DE)**
- **HEINRICH, Timo**
  **64823 Gross-Umstadt (DE)**
- **HÖLZEMANN, Günter**
  **64342 Seeheim-Jugenheim (DE)**
- **VAN AMSTERDAM, Christoph**
  **64295 Darmstadt (DE)**
- **BARTOSZYK, Gerd**
  **64331 Weiterstadt (DE)**
- **LEIBROCK, Joachim**
  **64319 Pfungstadt (DE)**
- **SEYFRIED, Christoph**
  **64342 Seeheim-Jugenheim (DE)**

(56) Entgegenhaltungen:
**US-A- 5 532 241 US-B1- 6 251 908**

EP 1 611 126 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft Chromenonindol-Derivate der Formel I

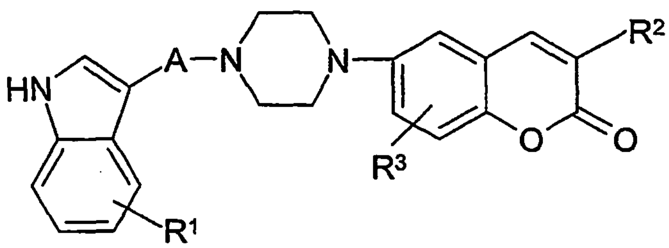

I

worin

- $R^1$ H, OH, CN, Hal, CONHR, OB, $CO_2B$ $CF_3$, $NR_2$, NRCOR, NRCOOR, $NRCONR_2$,
- $R^2$ $NR_2$, NRCOR, NRCOOR, $NRCONR_2$, $NO_2$, $NRSO_2R_2$, NRCSR, $NRCSNR_2$,
- $R^3$ H, OH, CN, Hal, CONHR, OB, $CO_2B$, $CF_3$, $NO_2$, $NR_2$, NRCOR, NRCOOR, $NRCONR_2$,
- R unabhängig voneinander H, B, Het, Ar,
- A eine geradkettige oder verzweigte, einfach oder mehrfach ungesättigte Kohlenstoffkette mit 2, 3, 4, 5, oder 6 C-Atomen,
- B ein geradkettiger oder verzweigter Alkylrest mit 1, 2, 3, 4, 5 oder 6 C-Atomen

bedeuten, sowie deren pharmazeutisch verwendbaren Solvate, Stereoisomere und Salze, wobei deren Mischungen in allen Verhältnissen umfasst sind.

Der Erfindung lag die Aufgabe zugrunde, neue Verbindungen aufzufinden, die eine hohe Bioverfügbarkeit aufweisen und in der Lage sind, den Serotoninspiegel im Gehirn deutlich zu erhöhen.

Es wurde gefunden, dass die Verbindungen der Formel I und ihre pharmazeutisch verwendbaren Solvate, Stereoisomere und Salze wertvolle pharmakologische Eigenschaften besitzen. Die Verbindungen der Formel I zeigen besondere Wirkungen auf das Zentralnervensystem, vor allem 5HT-Wiederaufnahme hemmende und 5 $HT_x$-agonistische und/oder -antagonistische Wirkungen, wobei unter $HT_x$ $HT_{1A}$, $HT_{1D}$, $HT_{2A}$ und/oder $HT_{2C}$ zu verstehen ist.

Verbindungen mit ähnlicher Struktur sind in der DE 197 30 989 beschrieben. Nun wurde festgestellt, dass eine Gruppe von bestimmten Chromenonindolen, und zwar solche, bei denen $R^2$ für die Reste $NR_2$, NRCOR, NRCOOR, $NRCONR_2$, $NO_2$, $NRSO_2R_2$ NRCSR oder $NRCSNR_2$ steht, eine im Vergleich zu anderen Chromenonindolen deutlich höhere Bioverfügbarkeit aufweisen und/oder einen deutlich höheren Serotoninspiegel im Gehirn bewirken (s. Abb. 1, 2 und 3). Somit sind die erfindungsgemäßen Verbindungen in Bezug auf die genannte Anmeldung als Auswahlerfindung zu betrachten.

Da die Verbindungen die Serotonin-Wiederaufnahme hemmen, eignen sie sich insbesondere als Antipsychotika, Neuroleptika, Antidepressiva, Anxiolytika und/oder Antihypertonika. Die Verbindungen zeigen serotoninagonistische und -antagonistische Eigenschaften. Sie hemmen die Bindung von tritiierten Serotoninliganden an hippocampale Rezeptoren (Cossery et al., European J. Pharmacol. 140 (1987), 143-155) und die synaptosomale Serotoninwiederaufnahme (Sherman et al., Life Sci. 23 (1978), 1863-1870). Außerdem treten Veränderungen der DOPA-Akkumulation im Striatum und der 5-HT-Akkumulation in verschiedenen Gehirnregionen auf (Seyfried et al., European J. Pharmacol. 160 (1989), 31-41). Die 5-$HT_{1A}$-antagonistische Wirkung wird in vitro z. B. durch Hemmung der durch 8-OH-DPAT-verursachten Aufhebung der elektrisch induzierten Kontraktion des Meerschweinchenileums nachgewiesen (Fozard und Kilbinger, Br. J. Pharmacol. 86 (1985) 601 P). Ex-vivo dient zum Nachweis der 5-$HT_{1A}$-antagonistischen Wirkung die Hemmung der durch 8-OH-DPAT verminderten 5-HTP-Akkumulation (Seyfried et al., European J. Pharmacol. 160 (1989), 31-41) und die Antagonisierung der durch 8-OH-DPAT- induzierten Effekte im Ultraschallvokalisationstest (DeVry, Psychpharmacol. 121 (1995), 1-26). Zum ex-vivo Nachweis der Serotoninwiederaufnahmehemmung kann die synaptosomale Aufnahmehemmung (Wong et al., Neuropsychopharmacol. 8 (1993), 23-33) und der p-Chloramphetaminantagonismus (Fulleret al., J. Pharmacol. Exp. Ther. 212 (1980), 115-119) herangezogen werden. Weiterhin treten analgetische und blutdrucksenkende Wirkungen auf.

Die Verbindungen sind daher geeignet für die Behandlung von Schizophrenie, kognitiven Defiziten, Angst, Depressionen, Übelkeit, tardiver Dyskinesien, Magen-Darm-Trakt-Störungen, Lernstörungen, altersabhängigen Erinnerungsstörungen, Psychosen und zur positiven Beeinflussung von Zwangsverhalten (OCD) und Essstörungen (z. B. Bulimie). Sie zeigen Wirkungen auf das Zentralnervensystem, vor allem zusätzliche 5-$HT_{1A}$-agonistische und 5-HT-Reuptake hemmende Wirkungen. Ebenso eignen sie sich zur Prophylaxe und zur Bekämpfung der Folgen cerebraler Infarktgeschehen (apoplexia cerebri) wie Schlaganfall und cerebraler Ischämien sowie zur Behandlung extrapyramidal-motorischer Nebenwirkungen von Neuroleptika sowie des Morbus Parkinson.

Die Verbindungen der Formel I eignen sich daher sowohl in der Veterinärals auch in der Humanmedizin zur Behandlung von Funktionsstörungen des Zentralnervensystems sowie von Entzündungen. Sie können zur Prophylaxe und zur Bekämpfung der Folgen cerebraler Infarktgeschehen (apoplexia cerebri) wie Schlaganfall und cerebraler Ischämien sowie zur Behandlung extrapyramidal-motorischer Nebenwirkungen von Neuroleptika sowie des Morbus Parkinson, zur akuten und symptomatischen Therapie der Alzheimer Krankheit sowie zur Behandlung der amyotrophen Lateralsklerose verwendet werden. Ebenso eignen sie sich als Therapeutika zur Behandlung von Hirn- und Rückenmarkstraumata. Sind sie jedoch auch geeignet als Arzneimittelwirkstoffe für Anxiolytika, Antidepressiva, Antipsychotika, Neuroleptika, Antihypertonika und/oder zur positiven Beeinflussung von Zwangsverhalten, Schlafstörungen, tardiver Dyskinesien, Lemstörungen, altersabhängiger Erinnerungsstörungen, Essstörungen wie Bulimie und/oder Sexualfunktionsstörungen.

Gegenstand der Erfindung sind bevorzugt Verbindungen der Formel I, worin der Rest $R^1$ für CN oder Hal steht, wobei CN bevorzugt ist und R, $R^2$, $R^3$, A und B die vor- und nachstehend für die Verbindungen der Formel I angegebenen Bedeutungen haben.

Weiterhin sind Verbindungen der Formel I bevorzugt, worin der Rest $R^3$ für H steht, wobei R, $R^1$, $R^2$, A und B die vor- und nachstehend für die Verbindungen der Formel I angegebenen Bedeutungen haben.

Ebenfalls sind Verbindungen der Formel I bevorzugt, worin der Rest $R^2$ für NRCOR oder NRCOOR steht, wobei R unabhängig voneinander H, B, Het oder Ar sein kann und $R^1$, $R^3$, A und B die vor- und nachstehend für die Verbindungen der Formel I angegebenen Bedeutungen haben.

Bevorzugt sind auch diejenigen Verbindungen der Formel I, worin A für $(CH_2)_m$ steht, wobei m = 2, 3, 4, 5 oder 6, besonders bevorzugt 4 steht und R, $R^1$, $R^2$, $R^3$ und B die vor- und nachstehend für die Verbindungen der Formel I angegebenen Bedeutungen haben.

Besonders bevorzugt sind die Verbindungen der Formel I, worin $R^1$ für CN oder Hal steht, wobei CN bevorzugt ist und $R^3$ für H steht, wobei R, $R^2$, A und B die vor- und nachstehend für die Verbindungen der Formel I angegebenen Bedeutungen haben.

Ganz besonders bevorzugt sind diejenigen Verbindungen der Formel I, worin $R^1$ für CN, $R^3$ für H und A für $(CH_2)_m$ steht, wobei m = 4 ist und R, $R^2$ und B die vor- und nachstehend für die Verbindungen der Formel I angegebenen Bedeutungen haben.

In besonders bevorzugten Ausführungsformen ist der Indolrest in 5-Stellung, ferner auch in der 6- oder 7-Stellung durch $R^1$ substituiert.

In einer ganz besonders bevorzugten Ausführungsform der vorliegenden Erfindung sind die Verbindungen der Formel I ausgewählt unter den folgenden Teilformeln I a bis I e.

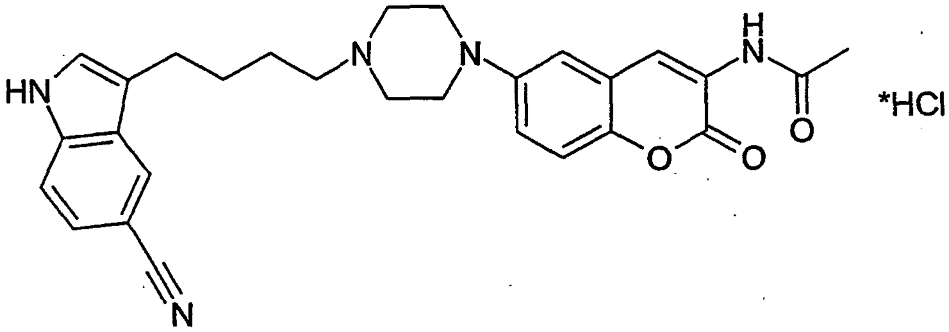

a

N-(6-{4-[4-(5-Cyano-1H-indol-3-yl)butyl]-piperazin-1-yl}-2-oxo-2H-chromen-3-yl)-methylamid (HCl) (EMD 391987)

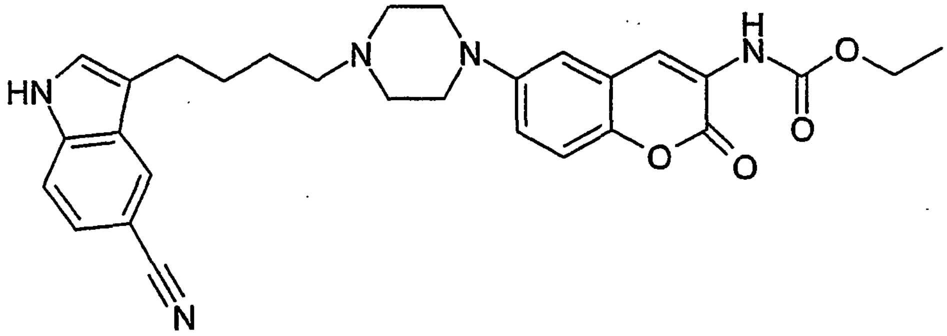

b

(6-{4-[4-(5-Cyano-1H-indol-3-yl)-butyl]-piperazin-1-yl}-2-oxo-2H-chromen-3-yl)-carbamidsäureethylester (EMD 480247)

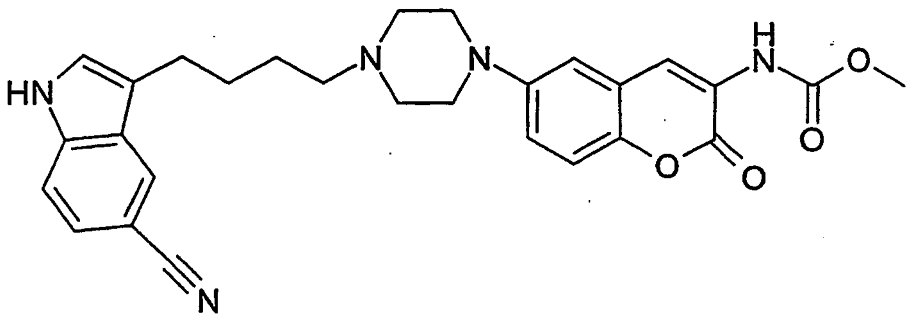

c

N-(6-{4-[4-(5-Cyano-1H-indol-3-yl)-butyl]-piperazin-1-yl}-2-oxo-2H-chromen-3-yl)-carbamidsäuremethylester (EMD 487535)

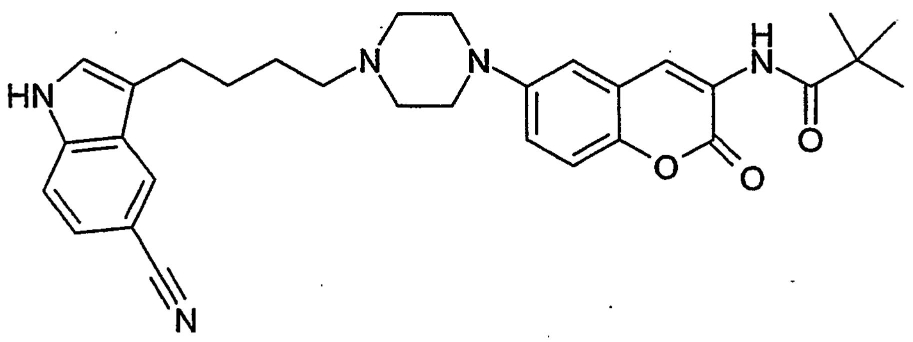

d

N-(6-{4-[4-(5-Cyano-1H-indol-3-yl)-butyl]-piperazin-1-yl}-2-oxo-2H-chromen-3-yl)-2,2-dimethyl-propionamid (EMD 480248)

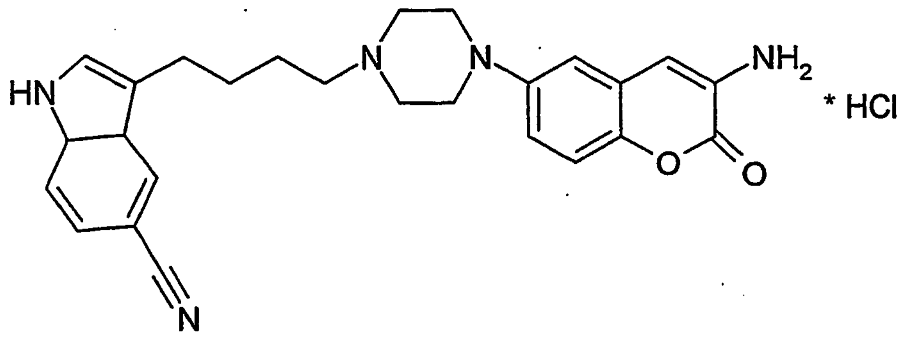

e

3-{4-[4-(3-Amino-2-oxo-2H-chromen-6-yl)-piperazin-1-yl]-butyl}-1H-indol-5-carbonitril (HCl) (EMD 480246)

Für alle Reste, die mehrfach auftreten, wie z. B. R oder B, gilt, dass deren Bedeutungen unabhängig voneinander sind.

Der Rest B bedeutet Alkyl und hat 1, 2, 3, 4, 5 oder 6, insbesondere 1, 2, 3 oder 4 C-Atome. Alkyl bedeutet einen linearen oder verzweigten Alkylrest, vorzugsweise einen unverzweigten Alkylrest und ein- oder mehrfach mit Halogen (Hal), z. B. perfluoriert, sein kann. Wenn ein Alkylrest mit Halogen substituiert ist, weist er vorzugsweise, abhängig von der Anzahl der Kohlenstoffatome des Alkylrests, 1, 2, 3, 4 oder 5 Halogenatome auf. Wenn ein Alkylrest mit Halogen substituiert ist, weist er vorzugsweise, abhängig von der Anzahl der Kohlenstoffatome des Alkylrests, 1, 2, 3, 4 oder 5 Halogenatome auf. Beispiele für Alkylgruppen sind daher Methyl, Ethyl oder Isopropyl, weiterhin n-Propyl, n-Butyl, sek.-Butyl oder tert.-Butyl, ferner auch Pentyl, 1-, 2- oder 3- Methylbutyl, 1,1-, 1,2- oder 2,2-Dimethylpropyl, 1-Ethylpropyl, Hexyl, 1-, 2-, 3- oder4-Methylpentyl, 1,1-, 1,2-, 1,3-, 2,2-, 2,3- oder 3,3- Dimethylbutyl, 1- oder 2-Ethylbutyl, 1-Ethyl-1-methylpropyl, 1-Ethyl-2- methylpropyl, 1,1,2- oder 1,2,2-Trimethylpropyl, ferner auch Fluormethyl, Difluormethyl, Trifluormethyl, 1,1,1-Trichlorethyl oder Pentafluorethyl.

Der Ausdruck "Aryl" umfasst einen unsubstituierten oder einfach oder mehrfach substituierten aromatischen mono-, bi- oder tricyclischen Kohlenwasserstoffrest wie zum Beispiel einen Benzolring oder Anthracen-, Phenanthren- oder Napthalen-Ringsysteme. Beispiele für geeignete Substituenten umfassen $NO_2$-, F-, Cl-, Br-, J-, HO-, $H_2N$-, $R^3HN$-, $(R^3)_2N$-, Alkyl-, Alkyl-O-, $CF_3$-O-, Alkyl-CO-, Aryl-, Aryl-O-, Aryl-CO-, Aryl-CONH-, $ArylSO_2$, $ArylSO_2$-HN-, wobei die Substituenten unabhängig voneinander 0 bis 5 mal vorkommen können.

Der Ausdruck "Het" umfasst einen unsubstituierten oder einfach oder mehrfach substituierten, gesättigten, ungesättigten oder aromatischen mono-, bi- oder tricyclischen heterocyclischen Rest. Als Heteroatome können S, N oder O ein- bis dreimal vorkommen. Beispiele für geeignete Substituenten umfassen $NO_2$-, F-, Cl-, Br-, J-, HO-, $H_2N$-, $R^3HN$-, $(R^3)_2N$-, Alkyl-, Alkyl-O-, $CF_3$-O-, Alkyl-CO-, Aryl-, Aryl-O-, Aryl-CO-, Aryl-CONH-, $ArylSO_2$-, $ArylSO_2$-HN-, wobei die Substituenten unabhängig voneinander 0 bis 5 mal vorkommen können.

Gegenstand der Erfindung ist ferner ein Verfahren zur Herstellung von den Verbindungen der Formel I sowie deren pharmazeutisch verwendbaren Prodrugs, Derivate, Solvate, Stereoisomere und Salze, **dadurch gekennzeichnet, dass** man eine Verbindung der Formel II

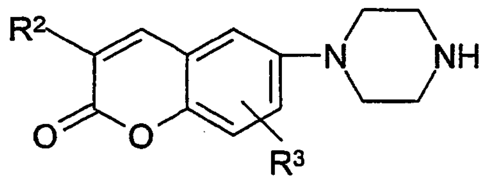

II

worin $R^2$ und $R^3$ die vor- und nachstehend für die Verbindungen der Formel I angegebenen Bedeutungen haben, mit einer Verbindung der Formel III umsetzt,

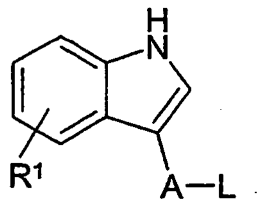

III

worin $R^1$ und A die vor- und nachstehend für die Verbindungen der Formel I angegebenen Bedeutungen haben und L für Cl, Br, I, OH oder eine reaktionsfähig veresterte OH-Gruppe oder eine andere leicht nucleophil substituierbare Abgangsgruppe wie Alkylsulfonyloxy mit 1-6 C-Atomen (z. B. Methansulfonyloxy) oder Arylsulfonyloxy mit 6-10 C-Atomen (z. B. Benzolsulfonyloxy, p-Toluolsulfonyloxy, 1- oder 2- Naphthalinsulfonyloxy), bedeutet, wobei I bevorzugt ist.

Gegenstand der vorliegenden Erfindung ist ebenfalls ein Verfahren zur Herstellung von den Verbindungen der Formel I sowie deren pharmazeutisch verwendbaren Prodrugs, Derivate, Solvate, Stereoisomere und Salze, **dadurch gekennzeichnet, daß** man in einer Michael-analogen Reaktion eine Verbindung der Formel IV,

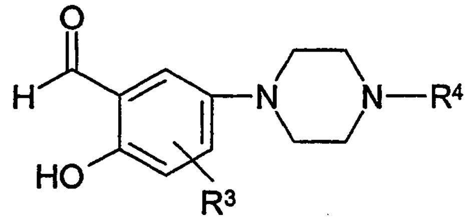

IV

worin $R^3$ die vor- und nachstehend für die Verbindungen der Formel I angegebenen Bedeutungen haben und $R^4$ für eine Aminoschutzgruppe oder H steht, mit Nitroessisäureethylester und Diethylammoniumchlorid und anschließender Reduktion der Nitrogruppe zur Verbindung der Formel V umsetzt

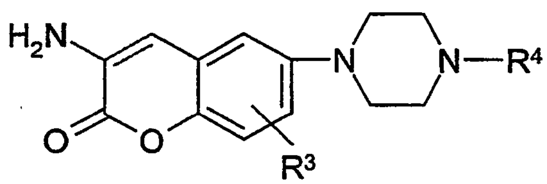

V

und die Verbindung der Formel V mit einer der Formel III entsprechenden Verbindung umsetzt.

Die Herstellung der Verbindungen der Formel I verfolgt im übrigen nach an sich bekannten Methoden, wie sie z.B. im Houben-Weyl (Methoden der Organischen Chemie, Georg Thieme Verlag, Stuttgart; Organic Reactions, John Wiley & Sons, Inc., New York) oder in der DE 197 30 989 beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, dass man sie aus dem Reaktionsgemisch nicht isoliert, sondern sofort weiter zu den Verbindungen der Formel I umsetzt.

Die Piperazin-Derivate der Formel II sind größtenteils bekannt. Sofern sie nicht käuflich zu erwerben oder bekannt sind, können sie nach an sich bekannten Methoden hergestellt werden. Sie können beispielsweise durch Umsetzung von Bis-(2-chlorethyl)-amin oder Bis-(2-chlorethyl)-ammoniumchlorid mit Amino-substituierten Benzopyranverbindungen hergestellt werden.

Die Indol-Derivate der Formel III sind größtenteils bekannt und teilweise auch käuflich zu erwerben. Ferner kann man die Verbindungen durch elektrophile oder in bestimmten Fällen auch nukleophile aromatische Substitutionen aus bekannten Verbindungen herstellen. Als Ausgangssubstanz dient vorzugsweise eine entsprechende Indol-3-alkansäure (herstellbar analog einer Japp-Klingemann-Typ Fischer Indolsynthese, vgl. dazu Böttcher et al., J. Med. Chem.

1992, 35, 4020-4026 oder Iyer et al., J. Chem. Soc. Perkin Trans. II 1973, 872-878).

Primäre Alkohole der Formel III in denen L eine OH-Gruppe darstellt sind z. B. durch Reduktion der entsprechenden Carbonsäuren oder ihrer Ester erhältlich. Behandlung mit Thionylchlorid, Bromwasserstoff, Phosphortribromid oder ähnlichen Halogenverbindungen liefert die entsprechenden Halogenide der Formel III in denen L ein Halogen darstellt. Die entsprechenden Sulfonyloxyverbindungen sind erhältlich aus den Alkoholen durch Umsetzung mit den entsprechenden Sulfonsäurechloriden. Die Iodverbindungen der Formel III (L = I) sind z. B. durch Einwirkung von Kaliumiodid auf die zughörigen p-Toluolsulfonsäureester oder entsprechenden Chloriden erhältlich.

Die Ausgangsstoffe der Formeln IV sind teilweise bekannt. Sofern sie nicht bekannt sind, können sie nach an sich bekannten Methoden hergestellt werden.

Die Umsetzung der Verbindungen II und III sowie V und III verläuft nach Methoden, wie sie für die Alkylierung bzw. Acylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z. B. Kohlenwasserstoffe, wie Benzol, Toluol, Xylol; Ketone wie Aceton, Butanon; Alkohole wie Methanol, Ethanol, Isopropanol, N-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses Piperazin-Derivates der Formel II kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°. Gegebenenfalls ist es notwendig, vor der Durchführung dieser Reaktionen weitere enthaltene Aminogruppen durch Einführung von geeigneten Schutzgruppen vor einer Alkylierung oder Acylierung zu schützen. Der Ausdruck Aminoschutzgruppe ist allgemein bekannt und bezieht sich auf Gruppen, die geeignet sind, eine Aminogruppe vor chemischen Umsetzungen zu schützen, die aber leicht entfernbar sind, nachdem die gewünschte chemische Reaktion an einer anderen Stelle des Moleküls durchgeführt worden ist. Da dem Fachmann solche Schutzgruppen sowie die Einführung und Abspaltung dieser aus zahlreichen Literaturstellen und Lehrbüchern wohl bekannt sind, muss an dieser Stelle darauf nicht näher eingegangen werden.

Es ist ferner möglich, eine Verbindung der Formel I zu erhalten, indem man ein Vorprodukt, das anstelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels. Reduzierbare (durch Wasserstoff ersetzbare) Gruppen sind insbesondere Sauerstoff in einer Carbonylgruppe, Hydroxyl, Arylsulfonyloxy (z. B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben angeführten Gruppen bzw. zusätzlichen Bindungen enthalten, reduktiv in eine Verbindung der Formel I überzuführen; dabei können gleichzeitig Substituenten in der Gruppe I, die in der Ausgangsverbindung enthalten sind, reduziert werden. Vorzugsweise bedient man sich hierzu des nascierenden Wasserstoffs oder komplexer Metallhydride, ferner der Reduktion nach Wolff-Kishner sowie der Reduktionen mit Wasserstoffgas unter Übergangsmetall-katalyse.

Wird als Reduktionsmittel nascierender Wasserstoff verwendet, so kann man diesen z. B. durch Behandlung von Metallen mit schwachen Säuren oder mit Basen erzeugen. So kann man z. B. ein Gemisch von Zink mit Alkalilauge oder von Eisen mit Essigsäure verwenden. Geeignet ist auch die Verwendung von Natrium oder einem anderen Alkalimetall gelöst in einem Alkohol wie Ethanol, Isopropanol, Butanol, Amyl- oder Isoamylalkohol oder Phenol. Man kann ferner eine Aluminium-NickelLegierung in alkalisch-wässriger Lösung, gegebenenfalls unter Zusatz von Ethanol, verwenden. Auch Natrium- oder Aluminiumamalgam in wässrigalkoholischer oder wässriger Lösung sind zur Erzeugung des nascierenden Wasserstoffs geeignet. Die Umsetzung kann auch in heterogener Phase durchgeführt werden, wobei man zweckmässig eine wässrige und eine Benzol- oder Toluol-Phase verwendet.

Als Reduktionsmittel können ferner besonders vorteilhaft komplexe Metallhydride, wie $LiAlH_4$, $NaBH_4$, Diisobutylaluminiumhydrid oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr. Als Lösungsmittel eignen sich hierfür insbesondere Ether wie Diethylether, Din-butylether, THF, Dioxan, Diglyme oder 1,2- Dimethoxyethan sowie Kohlenwasserstoffe wie Benzol. Für die Reduktion mit $NaBH_4$ sind in erster Linie Alkohole wie Methanol oder Ethanol, ferner Wasser sowie wässrige Alkohole als Lösungsmittel geeignet. Nach diesen Methoden reduziert man vorzugsweise bei Temperaturen zwischen -80 und +150°, insbesondere zwischen etwa 0 und etwa 100°.

Darüber hinaus ist es möglich, bestimmte Reduktionen durch Verwendung von $H_2$-Gas unter katalytischer Wirkung von Übergangsmetallen, wie z. B. Raney-Ni oder Pd durchzuführen. Man kann auf diese Weise z. B. Cl, Br, I, SH oder in bestimmten Fällen auch OH-Gruppen durch Wasserstoff ersetzen. Ebenso kann die $NH_2$-Gruppe in der Verbindung nach Formel V aus der Nitrogruppe durch katalytische Hydrierung mit $Pd/H_2$ in Methanol erhalten werden.

Verbindungen, die sonst der Formel I entsprechen, aber anstelle eines oder mehrerer H-Atome eine oder mehrere solvolysierbare Gruppe(n) enthalten, können zu den Verbindungen der Formel I solvolysiert, insbesondere hydrolysiert werden.

Weiterhin kann man eine Verbindung der Formel I nach an sich bekannten Methoden in eine andere Verbindung der Formel I umwandeln.

Verbindungen der Formel I, worin $R^1$ einen CONHR-Rest bedeutet, können durch Derivatisierung entsprechender substituierter Verbindungen der Formel I durch partielle Hydrolyse erhalten werden. Ferner ist es möglich, Cyan-substituierte Verbindungen der Formel I zunächst zu Säuren zu hydrolysieren und die Säuren mit primären oder sekundären Aminen zu amidieren. Bevorzugt ist die Umsetzung der freien Carbonsäure mit dem Amin unter den Bedingungen einer Peptidsynthese. Diese Reaktion gelingt vorzugsweise in Gegenwart eines Dehydratisierungsmittels, z. B. eines Carbodiimids wie Dicyclohexylcarbodümid oder N-(3-Dimethylaminopropyl)-N-ethyl-carbodiimid, ferner Propanphosphonsäureanhydrid (vgl. Angew. Chem. 92; 129 (1980)), Diphenylphosphorylazid oder 2-Ethoxy-N-ethoxycarbonyl-1,2-dihydrochinolin, in einem inerten Lösungsmittel, z. B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40°, vorzugsweise zwischen 0 und 30°. Anstelle der Säure bzw. des Amids können auch reaktionsfähige Derivate dieser Stoffe in die Reaktion eingesetzt werden, z. B. solche, in denen reaktive Gruppen intermediär durch Schutzgruppen blockiert sind. Die Säuren können auch in Form ihrer aktivierten Ester verwendet werden, die zweckmäßig in situ gebildet werden, z. B. durch Zusatz von 1-Hydroxybenztriazol oder N-Hydroxysuccinimid. So können auch beispielweise cyan-substituierte Indolreste zu Carboxyindol- oder Carboxamido-indolresten hydrolysiert werden.

Besonders günstig ist es aber auch in umgekehrter Weise, durch Wasserabspaltung, ausgehend von den Amiden, z. B. mittels Trichloracetylchlorid/$Et_3N$ [Synthesis (2), 184 (1985)] oder mit $POCl_3$ (J. Org. Chem. 26, 1003 (1961)), die Nitrile herzustellen.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z. B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure; Benzolsulfonsäure, p-Toluolsulfonsäure, Naphthalin-mono- und -disulfonsäuren, Lauryischwefeisäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen. In jenen Fällen, wo die Verbindungen der Formel I über freie Säuregruppen verfügen, kann durch Behandlung mit Basen ebenfalls eine Salzbildung erreicht werden. Als Basen eignen sich Alkalimetallhydroxide, Erdalkalimetallhydroxide oder organische Basen in Form von primären, sekundären oder tertiären Aminen.

Die pharmakologischen Eigenschaften der erfindungsgemäßen Verbindungen der Formel I wurden wie folgt geprüft:

Die Bioverfügbarkeit wurde nach einem in vielen Lehrbüchern der Pharmakokinetik beschriebenen Standardverfahren mit Hilfe der nach po und iv Verabreichung gemessenen AUC (Flächenintegral der Konzentration/Zeit-Kurven) bestimmt.

Für die Bestimmung der absoluten Bioverfügbarkeit wurde der Plasmakonzentrationsverlauf der genannten Substanzen nach intravenöser (iv) und oraler (po) Verabreichung an Wistar-Ratten (männlich; N=3 TiereNerabreichung) bestimmt. Folgendes Versuchsdesign wurde gewählt:

Verabreichungsweg: iv und po
Dosis: iv - 0.2 mg/kg; po - 0.5 mg/kg
Zeitpunkt der Blutentnahmen für die Bestimmung der Plasmakonzentrationen:

iv: 0.1, 0.5, 1, 2, 4, 6 und 24 h
po: 0.25, 0.5, 1, 2, 4, 6 und 24 h

Die Bestimmung der Plasmakonzentrationen erfolgte mit Hilfe der LC/MS/MS.

Mit Hilfe der Plasmakonzentrationen wurde die AUC (area under the curve bzw. Fläche unter der Konzentrations/Zeit-Kurve) nach der sog. Trapezformel berechnet.

Aus der AUC ergibt sich die Bioverfügbarkeit nach folgender Formel:

**Bioverfügbarkeit [%] = 100 x (AUC-po) / AUC-iv) / (Dosis-iv / Dosis-po)**

Diese Messung ergab für die in der DE 197 30 989 beschriebenen Verbindung 3-(4-(4-(2-Oxo-2H-1-benzopyran-6-yl)-1-piperazinyl)-butyl)-indol-5-carbonitril (EMD 135894) eine Bioverfügbarkeit von 10 %. Im Gegensatz dazu lag die Bioverfügbarkeit der Verbindung (6-{4-[4-(5-Cyano-1H-indol-3-yl)-butyl]-piperazin-1-yl}-2-oxo-2H-chromen-3-yl)-carbamidsäureethylester (s. Formel I b; EMD 480247) deutlich höher, nämlich bei 20 %.

Zur Bestimmung der Wirkung der Substanzen auf den kortikalen Serotoninspiegel der Ratte in vivo wurde eine Mikrodialysesonde mit einer semipermeablen Membran in das Himgewebe implantiert und mit einer der Zusammensetzung der Cerebrospinalflüssigkeit angepassten Lösung perfundiert (siehe dazu z. B. Di Chiara, Trends Pharmacol. Sci. 11, 117 - 121, 1990). Die Moleküle des extrazellulären Raumes wie z. B. auch der Neurotransmitter Serotonin diffundieren entsprechend ihrem Konzentrationsgradienten über diese Membran in das Innere der Sonde. Von dort werden Sie mit dem Perfusionsstrom zu einem Sammelgefäß transportiert. Im so gewonnenen Dialysat wird anschließend mit hochempfindlichen Analysemethoden die Serotoninkonzentration bestimmt. Die in regelmäßigen Abständen (von z. B. 15-20 Minuten) gewonnenen Fraktionen reflektieren damit die Konzentrationsverläufe der betrachteten Transmitter im Hirngewebe über einen Zeitraum von mehreren Stunden. Zuerst wird gemessen, wie hoch die Konzentration des Serotonins im Dialysat ohne Beeinflussung ist. Danach wird die zu testende Substanz appliziert und die Serotoninkonzentrationen vor und nach der Gabe der Substanzen verglichen.

Wie aus Abb. 1 zu ersehen ist, führt die Gabe von der Verbindung EMD 391987 (s. Formel I a) zu einem.wesentlich höheren Serotoninspiegel im

Gehirn wie die Gabe der gleichen Konzentration (1 mg/kg i.p.) der in der DE 197 30 989 beschriebenen und als als Vergleichssubstanz gewählten Verbindung 3-(4-(4-(2-Oxo-2H-1-benzopyran-6-yl)-1-piperazinyl)-butyl)-indol-5-carbonitril (EMD 135894).

Auch das in der EP 0 648 767 B1 beschriebene und bereits in klinischer Prüfung befindliche Vilazodon (5-{4-[4-(5-Cyano-3-inolyl)butyl]-1-piperanzinyl}-benzofuran-2-carboxamid; EMD 68843) wurde als Vergleichsverbindung herangezogen, da es ebenfalls über 5HT-Wiederaufnahme hemmende und 5 $HT_x$-agonistische und/oderantagonistische Eigenschaften verfügt. Auch gegenüber dieser Verbindung, die eine vergleichbare Konzentrationserhöhung an Serotonin in Gehirn bewirkt wie EMD 135894, führt die EMD 391987 zu einem deutlich höheren Serotoninspiegel im Gehirn.
Wie Abbildung 2 zeigt, führt eine weitere erfindungsgemäße Substanz nach Formel I, das 3-{4-[4-(3-Amino-2-oxo-2H-chromen-6-yl)-piperazin-1-yl]-butyl}-1H-indol-5-carbonitril (EMD 480246, s. Formel I e) zu einem noch höheren Serotoninspiegel als das bereits den Vergleichsverbindungen EMD 68843 und EMD 135894 überlegene EMD 391987.
Auch der (6-{4-[4-(5-Cyano-1H-indol-3-yl)-butyl]-piperazin-1-yl}-2-oxo-2H-chromen-3-yl)-carbamidsäureethylester (EMD 480247, s. Formel I b) zeigt eine dem Vilazodon (EMD 68843) deutlich überlegen Wirkung (s. Abb. 3).

Daraus ergibt sich, dass die erfindungsgemäßen Verbindungen der Formel I den anderen in der DE 197 30 989 und den in der EP 0 648 767 B1 1 beschriebenen Verbindungen bezüglich der Bioverfügbarkeit und der Serotoninspiegelerhöhung bzw. Serotonin-Wiederaufnahmehemmung deutlich überlegen sind.

Gegenstand der Erfindung ist ferner die Verwendung der Verbindung der Formel I und ihrer pharmazeutisch verwendbaren Prodrugs, Derivate, Solvate, Stereoisomere und Salze, wobei deren Mischungen in allen

Verhältnissen umfasst sind, zur Herstellung pharmazeutischer Zubereitungen, insbesondere auf nicht-chemischem Wege. Hierbei können sie z.B. zusammen mit einem festen, flüssigen oder halbflüssigen - Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer pharmazeutisch verwendbaren Prodrugs, Derivate, Solvate, Stereoisomere und Salze. Diese Zubereitungen können als Arzneimittel in der Human- und Veterinärmedizin eingesetzt werden. Als Trägersubstanzen kommen organische oder anorganische Stoffe in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositoren, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenden Lyophilisate z. B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Die Verbindungen der Formel I und ihre pharmazeutisch verwendbaren Prodrugs, Derivate, Solvate, Stereoisomere und Salze können bei der therapeutischen Behandlung des menschlichen oder tierischen Körpers und bei der Bekämpfung von Krankheiten verwendet werden. Sie eignen sich zur Behandlung von Erkrankungen des Zentralnervensystems wie Spannungszuständen, Depressionen, Angstzuständen, Schizophrenie, Magen-Darm-Trakt-Störungen, Übelkeit, tardiven Dyskinesien, Parkinsonismus und/oder Psychosen und von Nebenwirkungen bei der Behandlung der Hypertonie (z. B. mit $\alpha$- Methyldopa). Ferner können die Verbindungen in der Endokrinologie und Gynäkologie Verwendung finden, z. B. zur Therapie von Akromegalie, Hypogonadismus, sekundärer Amenorrhoe, prämenstruellem Syndrom, unerwünschter puerperaler Laktation, weiterhin zur Prophylaxe und Therapie cerebraler Störungen (z. B. Migräne), insbesondere in der Geriatrie ähnlich wie gewisse Ergot-Alkaloide.

Insbesondere bevorzugt können sie auch als Therapeutika zur Bekämpfung der Folgen cerebraler Infarktgeschehen (apoplexia cerebri), wie Schlaganfall und cerebraler Ischämien und zur Behandlung von Hirn- und Rückenmarkstraumata eingesetzt werden.

Insbesondere sind sie jedoch geeignet als Arzneimittelwirkstoffe für Anxiolxytika, Antidepresiva, Antipsychotika, Neuroleptika, Antihypertonika und/oder zur positiven Beeinflussung von Zwangsverhalten (OCD), Schlafstörungen, tardiver Dyskinesien, Lernstörungen, altersabhängiger Erinnerungsstörungen, Essstörungen wie Bulimie und/oder Sexualfunktionsstörungen.

Dabei werden die erfindungsgemäßen Substanzen in der Regel in Analogie zu bekannten, im Handel befindlichen Präparaten (z. B. Citalopram und Fluoxetine) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen liegen zwischen etwa 0,2 und 500 mg, insbesondere zwischen 0,2 und 50 mg pro Dosierungseinheit. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

**Abbildungen:**

**Fig. 1:** Zeitabhängiger Verlauf der Serotoninkonentration (5- Hydroxytryptamin, 5-HT) im Dialysat vor und nach Applikation (ip) von EMD 135894, EMD 68843 und EMD 391987 (jeweilige Konzentration: 1 mg/kg Körpergewicht).

**Fig. 2:** Zeitabhängiger Verlauf der Serotoninkonentration (5- Hydroxytryptamin, 5-HT) im Dialysat vor und nach Applikation (ip) EMD 480246 und EMD 391987 (je 0.3 mg/kg Körpergewicht).

**Fig. 3:** Zeitabhängiger Verlauf der Serotoninkonentration (5- Hydroxytryptamin, 5-HT) im Dialysat vor und nach Applikation (ip) von 0.3 mg/kg Körpergewicht bzw. 1.0 mg/kg Körpergewicht EMD 480247 im Vergleich zu Vilazodon (EMD 68843; 1 mg/kg Körpergewicht).

**Beispiele:**

**Beispiel 1**

Synthese von N-(6-{4-[4-(5-Cyano-1H-indol-3-yl)-butyl]-piperazin-1-yl}-2-oxo-2H-chromen-3-yl)-methylamid

a)

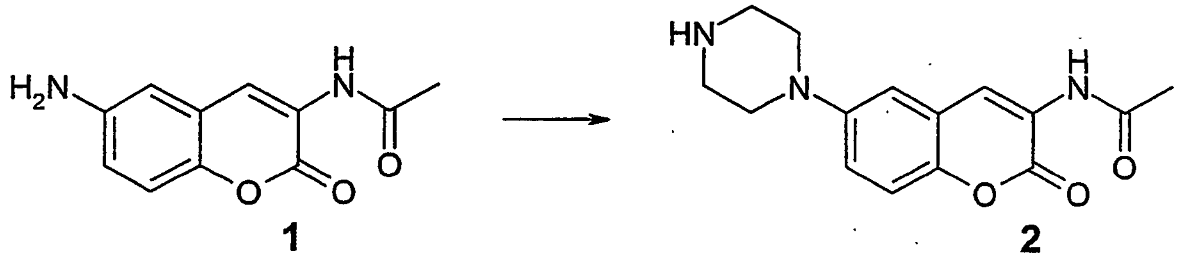

20,0 g (91,7 mmol) **1** wurden in Chlorbenzol (600 mL) vorgelegt, 16,4 g (91,7 mmol) Bis(2-chlorethyl)ammoniumchlorid und 12,7 g mg (92,0 mmol) Kaliumcarbonat zugefügt und 4 d unter Rückfluss erhitzt. Der Rückstand wurde abfiltriert, mit viel Wasser gewaschen und im
Vakuumtrockenschrank getrocknet.
Ausbeute: 14 g farblose Festsubstanz **(2).**
$[M+H]^+$ 288 (HPLC-MS)

b)

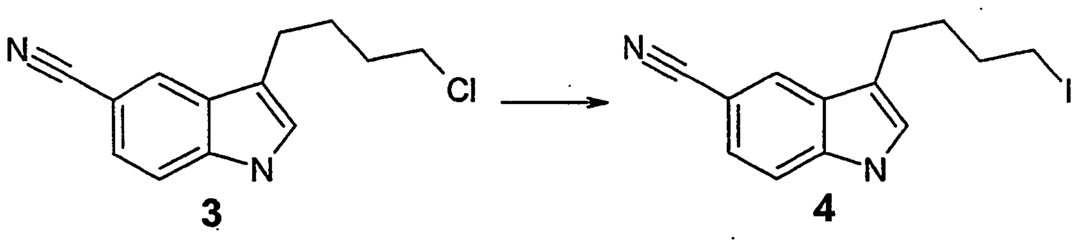

Es wurde **3** (150 g, 0,65 mol) in 500 ml Aceton (getr.) gelöst und Natriumiodid (580 g, 3,87 mol) eingerührt. Die Suspension wurde bei RT gerührt. Nach 118 Stunden wurden weitere 18 g NaI (0,12 mol) zugegeben und bei RT weitere 3 d gerührt. Die Suspension wurde abgesaugt, mit Aceton (getr.) nachgewaschen und mit 100 g NaI (gemörsert) versetzt und bei RT gerührt. Nach 24 h wurden nochmals 100 g NaI gemörsert zugegeben und weitere 72 h gerührt. Anschließend wurden erneut 100 g NaI (gemörsert) zugegeben und weitere 5 d gerührt. Die Suspension wurde abgesaugt mit Aceton gut nachgewaschen, der Filterkuchen verworfen und das Filtrat zum Rückstand eingeengt. Der Rückstand wurde mit Wasser verrührt und mit Essigester ausgeschüttelt, die Essigesterlösung getrocknet, filtriert und zum Rückstand eingeengt. Der Rückstand wurde mit einem Gemisch aus 300 ml Petrolether und 200 ml Diethylether verrührt und abgesaugt. Auf der Nutsche wurde mit 50 ml kaltem Diethylether nachgewaschen und die Kristalle an der Luft getrocknet.
Ausbeute: 184 g bräunliche Kristalle **(4)**
$[M+H]^+$ 325 (HPLC-MS)

c)

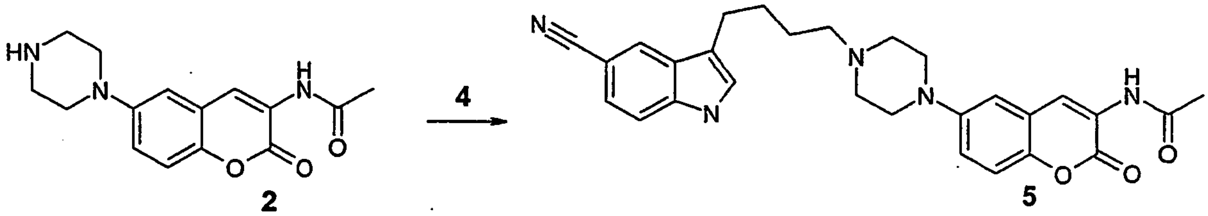

12,4 g (43,0 mmol) **2** wurde in *N*-Methylpyrrolidon (150 mL) aufgenommen, 13,9 g (43,0 mmol) **4** und 11,1 g (85,8 mmol)

Diisopropyldiethylamin zugegeben und 3 Tage bei 140°C gerührt. Die Reaktionslösung wurde auf Eiswasser geschüttet und der ausgefallene braune Niederschlag abfiltriert. Der Rückstand wurde säulenchromato-graphisch (Essigsäureethylester/MeOH, 9 : 1) aufgereinigt und die nahezu saubere

Fraktion aus tert-Butylmethylether/Hexan umkristallisiert. Der Feststoff wurde auf übliche Weise in das Hydrochlorid überführt.
Ausbeute: 3,8 g farblose Festsubstanz, HCl-Salz **(5)**
$[M+H]^+$ 484 (HPLC-MS)

**Beispiel 2**

Synthese von N-(6-{4-[4-(5-Cyano-1H-indol-3-yl)-butyl]-piperazin-1-yl}-2-oxo-2H-chromen-3-yl)-ethylamid

a)

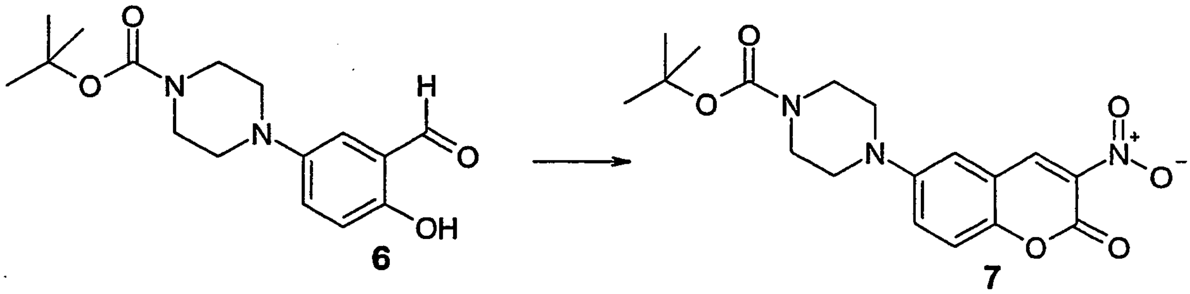

6 7

**6** (20,0 g, 65,1 mmol), Nitroessigsäureethylester (9,00 mL, 78,3 mmol) und Diethylammoniumchlorid (8,58 g, 78,3 mmol) wurden in Toluen (500 mL) 3 d am Wasserabscheider erhitzt. Die abgekühlte Reaktionslösung wurde mit Wasser gewaschen, getrocknet, vom Trockenmittel filtriert und das Lösungsmittel entfernt. Der Rückstand wurde säulenchromatographisch aufgereinigt (Essigsäureethylester/Cyclohexan).
Ausbeute: 13,2 g gelbliche Festsubstanz **(7)**
$[M+H]^+$ 376, $[M-55]^+$ 320 (HPLC-MS)

b)

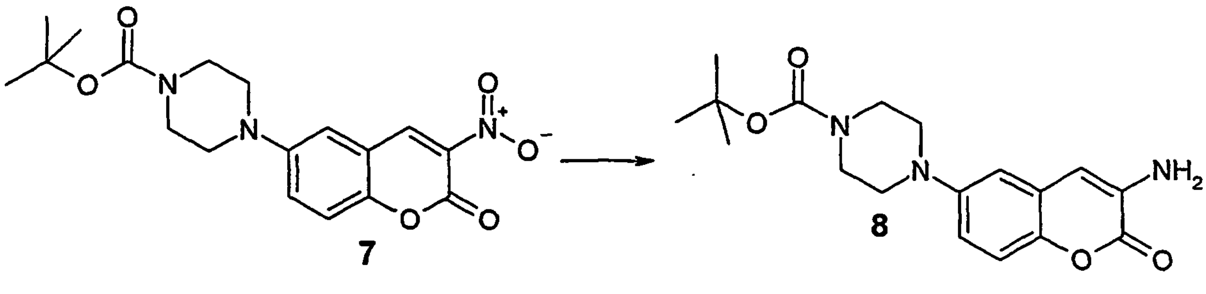

7 8

**7** (3,00 g, 7,99 mmol) wurde in MeOH (50 mL) gelöst, Pd-C.5% (1 g) zugegeben und 24 h in einer Wasserstoffatmosphäre gerührt. Der Katalysator wurde abfiltriert und das Filtrat zur Trockne eingedampft. Der Rückstand wurde säulenchromatographisch aufgereinigt (Essigsäureethylester/Methanol).
Ausbeute: 1,50 g bräunliche Festsubstanz **(8)**
$[M+H]^+$ 346, $[M-55]^+$ 290 (HPLC-MS)

c)

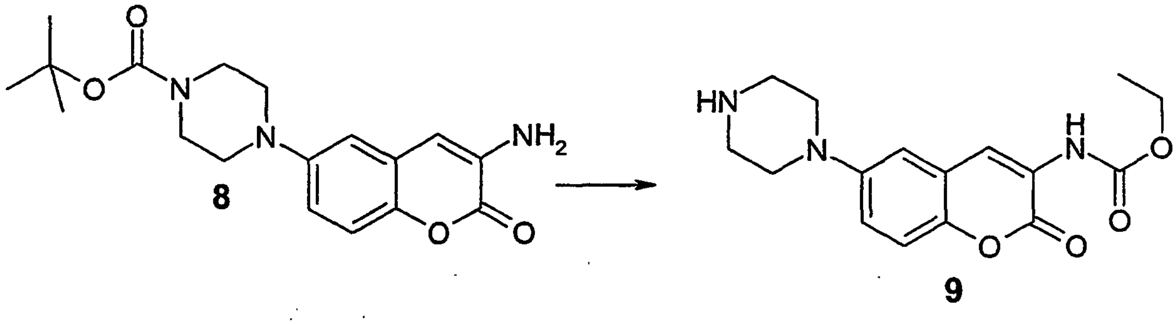


**8** (375 mg, 1,09 mmol) wurde in Dichlormethan (10 mL) gelöst, Pyridin(0,11 mL, 1,1 mmol) zugegeben und auf 0°C abgekühlt. Bei dieser Temperatur wurde Ethylchlorformiat (141 mg, 1,10 mmol) zugetropft und über Nacht bei Raumtemperatur nachgerührt. Der Ansatz wurde eingedampft und säulenchromatographisch aufgereinigt (Essigsäureethyl-ester/Cyclohexan, 1:1).
Ausbeute: 320 mg farblose Festsubstanz
$[M+H]^+$ 418, $[M-55]^+$ 362 (HPLC-MS)
Die so erhaltene Substanz wurde in gesättigter ethanolischer HCl-Lösung (10 mL) 60 min bei Raumtemperatur gerührt und das Lösungsmittel abgezogen.
Ausbeute: 270 mg farblose Festsubstanz, Di-HCl-Salz **(9)**
$[M+H]^+$ 355 (HPLC-MS)

d)

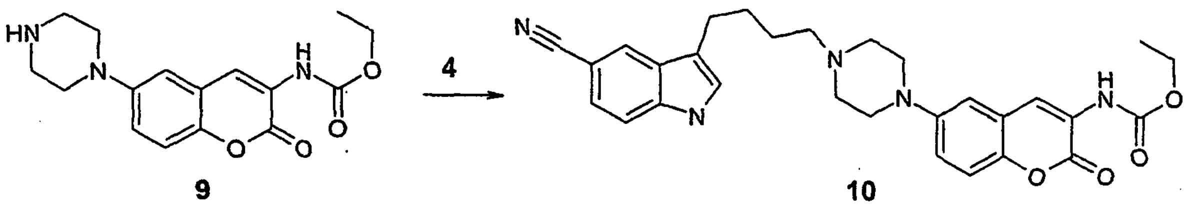


**9** (200 mg, 0,51 mmol), **4** (s. Beispiel 1; 166 mg, 0,51 mmol) und Diisopropylethylamin (0,26 mL, 1,53 mmol) wurden in Acetonitril (5 mL) 18 h unter Rückfluss gekocht. Das Lösungsmittel wurde entfernt und der Rückstand und säulenchromatographisch aufgereinigt (Essigsäureethylester/Methanol). Der Feststoff wurde auf übliche Weise in das Hydrochlorid überführt.
Ausbeute: 70 mg farblose Festsubstanz, HCl-Salz **(10)**
$[M+H]^+$ 514 (HPLC-MS)

**Beispiel 3**

Synthese von N-(6-{4-[4-(5-Cyano-1H-indol-3-yl)-butyl]-piperazin-1-yl}-2-oxo-2H-chromen-3-yl)-amin

a)

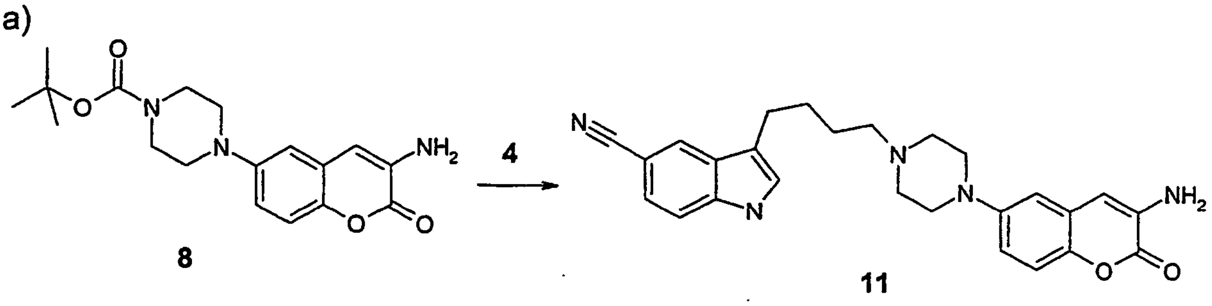

Die Verbindung **8** wurde analog der Stufen a) und b) in Beispiel 2 hergestellt. Anschließend wurde **8** (1,00 g, 2,90 mmol) in gesättigter ethanolischer HCl-Lösung (10mL) 60 min bei Raumtemperatur gerührt und das Lösungsdmittel abgezogen.
Ausbeute: 880 mg farblose Festsubstanz, Di-HCl-Salz **(8)**
$[M+H]^+$ 246 (HPLC-MS)

Die so gewonnene Verbindung **8** (50 mg, 0,16 mmol) wurde mit **4** (77 mg, 0,24 mmol), Diisopropylethylamin (0,08 mL, 0,47 mmol) in. Acetonitril (2 mL) 18 h unter Rückfluss gekocht. Das Lösungsmittel wurde entfernt und der Rückstand und säulenchromatographisch aufgereinigt (Essigsäureethylester/Methanol). Der Feststoff wurde auf übliche Weise in das Hydrochlorid überführt.
Ausbeute: 25 mg farblose Festsubstanz, HCl-Salz **(11)**
$[M+H]^+$ 442 (HPLC-MS)

**Beispiel A: Suppositorien**

Man schmilzt ein Gemisch von 20 g eines Wirkstoffes der Formel I mit 100 g Sojalecithin und 1400 g Kakaobutter, giesst in Formen und lässt erkalten. Jedes Suppositorium enthält 20 mg Wirkstoff.

**Beispiel B: Lösung**

Man bereitet eine Lösung aus 1 g eines Wirkstoffes der Formel I, 9. 38 g $NaH_2PO_4 \times 2\ H_2O$, 28.48 g $NaH_2PO_4 \times 12\ H_2O$ und 0.1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

**Beispiel C: Salbe**

Man mischt 500 mg eines Wirkstoffes der Formel I mit 99,5 g Vaseline unter aseptischen Bedingungen.

**Beispiel D: Tabletten**

Ein Gemisch von 1 kg Wirkstoff der Formel I, 4 kg Lactose, 1.2 kg Kartoffelstärke, 0.2 kg Talk und 0.1 kg Magnesiumstearat wird in üblicher Weise zu Tabletten verpresst, derart, daß jede Tablette 10 mg Wirkstoff enthält.

**Beispiel E: Dragees**

Analog Beispiel D werden Tabletten gepresst, die anschließend in üblicher Weise mit einem Überzug aus Saccharose, Kartoffelstärke, Talk, Tragant und Farbstoff überzogen werden.

**Beispiel F: Kapseln**

2 kg Wirkstoff der Formel I werden in üblicher Weise in Hartgelatinekapseln gefüllt, so daß jede Kapsel 20 mg des Wirkstoffs enthält.

**Beispiel G: Ampullen**

Eine Lösung von 1 kg Wirkstoff der Formel I in 60 l zweifach destilliertem Wasser wird in Ampullen abgefüllt,

unter aseptischen Bedingungen lyophilisiert und steril verschlossen. Jede Ampulle enthält 10 mg Wirkstoff.

**Patentansprüche**

1. Verbindungen der Formel I

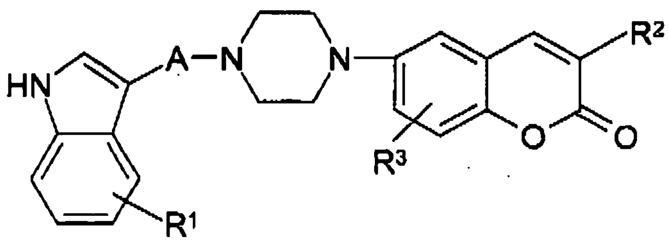

I

worin

$R^1$ H, OH, CN, Hal, CONHR, OB, $CO_2B$, $CF_3$, $NO_2$, $NR_2$, NRCOR, NRCOOR, $NRCONR_2$,
$R^2$ $NR_2$, NRCOR, NRCOOR, $NRCONR_2$, $NO_2$, $NRSO_2R_2$ NRCSR, $NRCSNR_2$,
$R^3$ H, OH, CN, Hal, CONHR, OB, $CO_2B$, $CF_3$, $NO_2$, $NR_2$, NRCOR, NRCOOR, $NRCONR_2$,
R unabhängig voneinander H, B, Het, Ar,
Het einen unsubstituierten oder einfach oder mehrfach substituierten, gesättigten, ungesättigten oder aromatischen mono-, bi- oder tricyclischen heterocyclischen Rest, wobei Heteroatome S, N oder O sein und ein- bis dreimal vorkommen können, mit Substituenten $NO_2$-, F-, Cl-, Br-, J-, HO-, $H_2N$-, $R^3HN$-, $(R^3)_2N$-, Alkyl-, Alkyl-O-, $CF_3$-O-, Alkyl- CO-, Aryl-, Aryl-O-, Aryl-CO-, Aryl-CONH-, $ArylSO_2$-, $ArylSO_2$-HN-, wobei die Substituenten unabhängig voneinander 0 bis 5 mal vorkommen können,
Ar einen unsubstituierten oder einfach oder mehrfach substituierten aromatischen mono-, bi- oder tricyclischen Kohlenwasserstoffrest, wobei geeignete Substituenten $NO_2$- F-, Cl-, Br-, J-, HO-, $H_2N$-, $R^3HN$-, $(R^3)_2N$- Alkyl-, Alkyl-O-, $CF_3$-O-, Alkyl-CO-, Aryl-, Aryl-O-, Aryl-CO-, Aryl- CONH-, $ArylSO_2$-, $ArylSO_2$-HN- sind und die Substituenten unabhängig voneinander 0 bis 5 mal vorkommen können,
A eine geradkettige oder verzweigte, einfach oder mehrfach ungesättigte Kohlenstoffkette mit 2, 3, 4, 5, oder 6 C-Atomen,
B ein geradkettiger oder verzweigter Alkylrest mit 1, 2, 3, 4, 5 oder 6 C- Atomen

bedeuten, sowie deren pharmazeutisch verwendbaren Solvate, Stereoisomere und Salze und deren Mischungen in allen Verhältnissen.

2. Verbindungen der Formel I nach Anspruch 1, **dadurch gekennzeichnet dass** der Rest $R^1$ für CN oder Hal, vorzugsweise CN steht.

3. Verbindungen der Formel I nach Anspruch 1 und/oder 2, **dadurch gekennzeichnet dass** der Rest $R^3$ für H steht.

4. Verbindungen der Formel nach einem oder mehreren der Ansprüche 1 bis 3, **dadurch gekennzeichnet dass** der Rest $R^2$ für NRCOR oder NRCOOR steht.

5. Verbindungen der Formel I nach einem oder mehreren der Ansprüche 1 bis 4, **dadurch gekennzeichnet dass** A für $(CH_2)_m$ steht, wobei m = 2, 3, 4, 5 oder 6, bevorzugt 4 ist.

6. Verbindungen der Formel I nach einem oder mehreren der Ansprüche 1 bis 5, **dadurch gekennzeichnet, dass** der Rest $R^1$ für CN oder Hal steht, wobei CN bevorzugt ist und $R^3$ für H steht.

7. Verbindungen der Formel I nach einem oder mehreren der Ansprüche 1 bis 6, **dadurch gekennzeichnet, dass** $R^1$ für CN, $R^3$ für H und A für $(CH_2)_m$ steht, wobei m = 4 ist.

8. Verbindungen der Formel I nach einem oder mehreren der Ansprüche 1 bis 7, **dadurch gekennzeichnet, dass**

der Rest $R^1$ an der Position 5 des Indolrestes steht.

9. Verbindungen der Formel I ausgewählt aus der Gruppe bestehend aus
N-(6-{4-[4-(5-Cyano-1H-indol-3-yl)-butyl]-piperazin-1-yl}-2-oxo-2H-chromen-3-yl)-methylamid,
(6-{4-[4-(5-Cyano-1H-indol-3-yl)-butyl]-piperazin-1-yl)-2-oxo-2H-chromen-3-yl)-carbamidsäureethylester,
N-(6-{4-[4-(5-Cyano-1H-indot-3-yl)-butyl]-piperazin-1-yl}-2-oxo-2H-chromen-3-yl)-carbamidsäuremethylester,
N-(6-{4-[4-(5-Cyano-1H-indol-3-yl)-butyl]-piperazin-1-yl}-2-oxo-2H-chromen-3-yl)-2,2-dimethyl-propionamid,
3-{4-[4-(3-Amino-2-oxo-2H-chromen-6-yl)-piperazin-1-yl]-butyl}-1H-indol-5-carbonitril,
sowie deren pharmazeutisch verwendbaren Prodrugs, Derivate, Solvate, Stereoisomere und Salze.

10. Verfahren zur Herstellung von Verbindungen der Formel I nach einem oder mehreren der Ansprüche 1 bis 9, **dadurch gekennzeichnet, dass** man eine Verbindung der Formel II

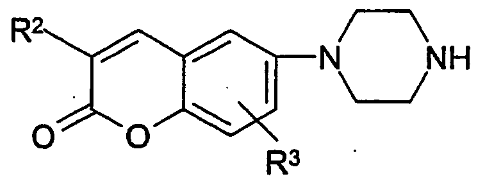

II

worin $R^2$ und $R^3$ die in Anspruch 1. angegebenen Bedeutungen haben, mit einer Verbindung der Formel III umsetzt,

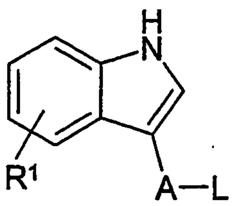

III

worin $R^1$ und A die in Anspruch 1 angegebenen Bedeutungen haben und L für Cl, Br, I; OH oder eine reaktionsfähig veresterte OH-Gruppe oder eine andere leicht nucleophil substituierbare Abgangsgruppe bedeutet.

11. Verfahren zur Herstellung von Verbindungen der Formel I nach einem oder mehreren der Ansprüche 1 bis 9, **dadurch gekennzeichnet, daß** man in einer Michael-analogen Reaktion eine Verbindung der Formel IV

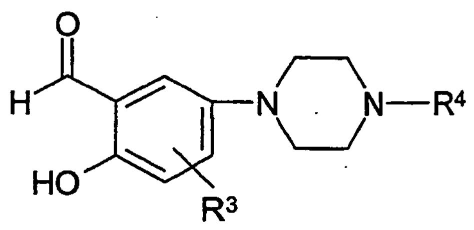

IV

worin $R^3$ die in Anspruch 1 angegebene Bedeutung hat und $R^4$ für eine Aminoschutzgruppe oder H steht mit Nitroessisäureethylester und Diethylammoniumchlorid und anschließender Reduktion der Nitrogruppe zur Verbindung der Formel V umsetzt.

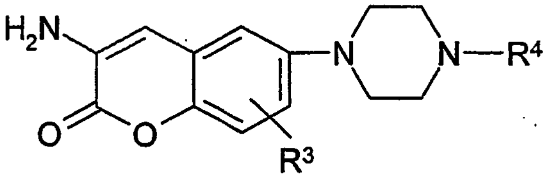

V

und die Verbindung der Formel V mit einer der Formel III entsprechenden Verbindung umsetzt.

**12.** Verbindungen der Formel I nach einem oder mehreren der Ansprüche 1 bis 7 als Arzneimittel.

**13.** Arzneimittel enthaltend eine wirksame Menge an einer Verbindung der Formel I nach einem odermehreren der Ansprüche 1 bis 9 neben gegebenenfalls einem oder mehreren inerten Trägerstoffen, Hilfsstoffen und/oder Verdünnungsmitteln.

**14.** Arzneimittel nach Anspruch 13, **dadurch gekennzeichnet, dass** mindestens ein weiterer Arzneimittelwirkstoff enthalten ist.

**15.** Verfahren zur Herstellung eines Arzneimittels gemäß Anspruch 13 oder 14, **dadurch gekennzeichnet, daß** auf nichtchemischem Wege eine Verbindung der Formel I nach einem oder mehreren der Ansprüche 1 bis 9 und gegebenenfalls ein weiterer Arzneimittelwirkstoff in einen oder mehrere inerte Trägerstoffe und/oder Verdünnungsmittel eingearbeitet wird.

**16.** Verwendung der Verbindungen der Formel I nach einem oder mehreren der Ansprüche 1 bis 9 zur Herstellung eines Medikaments zur Prophylaxe und/oder Therapie von Erkrankungen ausgewählt aus der Gruppe umfassend Depression, Schlaganfall, cerebrale Ischämien, extrapyramidal-motorischer Nebenwirkungen von Neuroleptika sowie des Morbus Parkinson, Alzheimer Krankheit, amyotrophe Lateralsklerose, Hirn- und Rückenmarkstraumata, Zwangsverhalten, Schlafstörungen, tardiver Dyskinesien, Lernstörungen, altersabhängiger Erinnerungsstörungen, Essstörungen wie Bulimie und/oder Sexualfunktionsstörungen.

**17.** Arzneimittelkit, bestehend aus getrennten Packungen von

a) einer wirksamen Menge einer Verbindung der Formel I nach einem oder mehreren der Ansprüche 1 bis 9 und
b) einer wirksamen Menge eines weiteren Arzneimittelwirkstoffes.

**Claims**

**1.** Compounds of the formula I

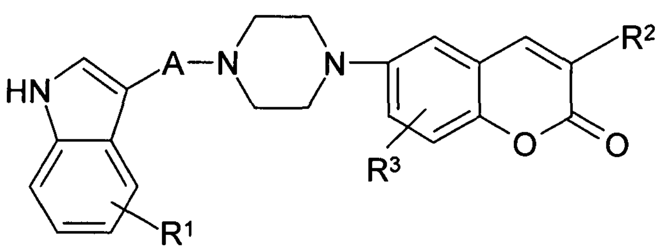

I

in which

$R^1$ denotes H, OH, CN, Hal, CONHR, OB, $CO_2B$, $CF_3$, $NO_2$, $NR_2$, NRCOR, NRCOOR, $NRCONR_2$,
$R^2$ denotes $NR_2$, NRCOR, NRCOOR, $NRCONR_2$, $NO_2$, $NRSO_2R_2$, NRCSR, $NRCSNR_2$,
$R^3$ denotes H, OH, CN, Hal, CONHR, OB, $CO_2B$, $CF_3$, $NO_2$, $NR_2$, NRCOR, NRCOOR, $NRCONR_2$,
R, independently of one another, denotes H, B, Het, Ar,

Het denotes an unsubstituted or mono- or polysubstituted, satu- rated, unsaturated or aromatic mono-, bi- or tricyclic heterocyclic radical, where heteroatoms can be S, N or O and may occur once to three times, with substituents $NO_2$-, F-, Cl-, Br-, I-, HO-, $H_2N$-, $R^3HN$-, $(R^3)_2N$-, alkyl-, alkyl-O-, $CF_3$-O-, alkyl-CO-, aryl-, aryl-O-, aryl-CO-, aryl-CONH-, aryl-$SO_2$-, aryl-$SO_2$-HN-, where the substituents may occur, independently of one another, 0 to 5 times,
Ar denotes an unsubstituted or mono- or polysubstituted aromatic mono-, bi- or tricyclic hydrocarbon radical, where suitable sub- stituents are $NO_2$-, F-, Cl-, Br-, I-, HO-, $H_2N$-, $R^3HN$-, $(R^3)_2N$-, alkyl-, alkyl-O-, $CF_3$-O-, alkyl-CO-, aryl-, aryl-O-, aryl-CO-, aryl- CONH-, aryl$SO_2$-, aryl$SO_2$-HN- and the substituents may occur, independently of one another, 0 to 5 times,
A denotes a straight-chain or branched, mono- or polyunsaturated carbon chain having 2, 3, 4, 5 or 6 C atoms,
B denotes a straight-chain or branched alkyl radical having 1, 2, 3, 4, 5 or 6 C atoms,

and pharmaceutically usable solvates, stereoisomers and salts thereof, and mixtures thereof in all ratios.

2. Compounds of the formula I according to Claim 1, **characterised in that** the radical $R^1$ stands for CN or Hal, preferably CN.

3. Compounds of the formula I according to Claim 1 and/or 2, **characterised in that** the radical $R^3$ stands for H.

4. Compounds of the formula I according to one or more of Claims 1 to 3, **characterised in that** the radical $R^2$ stands for NRCOR or NRCOOR.

5. Compounds of the formula I according to one or more of Claims 1 to 4, **characterised in that** A stands for $(CH_2)_m$, where m = 2, 3, 4, 5 or 6, preferably 4.

6. Compounds of the formula I according to one or more of Claims 1 to 5, **characterised in that** the radical $R^1$ stands for CN or Hal, where CN is preferred, and $R^3$ stands for H.

7. Compounds of the formula I according to one or more of Claims 1 to 6, **characterised in that** $R^1$ stands for CN, $R^3$ stands for H and A stands for $(CH_2)_m$, where m = 4.

8. Compounds of the formula I according to one or more of Claims 1 to 7, **characterised in that** the radical $R^1$ is in position 5 of the indole radical.

9. Compounds of the formula I selected from the group consisting of N-(6-{4-[4-(5-cyano-1H-indol-3-yl)butyl]piperazin-1-yl}-2-oxo-2H-chromen-3-yl)methylamide,
ethyl (6-{4-[4-(5-cyano-1H-indol-3-yl)butyl]piperazin-1-yl}-2-oxo-2H-chromen-3-yl)carbamate,
methyl N-(6-{4-[4-(5-cyano-1H-indol-3-yl)butyl]piperazin-1-yl}-2-oxo-2H-chromen-3-yl)carbamate,
N-(6-{4-[4-(5-cyano-1H-indol-3-yl)butyl]piperazin-1-yl}-2-oxo-2H-chromen-3-yl)-2,2-dimethylpropionamide,
3-{4-[4-(3-amino-2-oxo-2H-chromen-6-yl)piperazin-1-yl]butyl}-1H-indole-5-carbonitrile,
and pharmaceutically usable prodrugs, derivatives, solvates, stereoisomers and salts thereof.

10. Process for the preparation of compounds of the formula I according to one or more of Claims 1 to 9, **characterised in that** a compound of the formula II

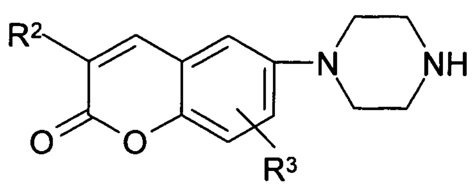

II

in which $R^2$ and $R^3$ have the meanings indicated in Claim 1, is reacted with a compound of the formula III

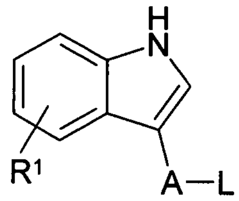

III

in which $R^1$ and A have the meanings indicated in Claim 1, and L stands for Cl, Br, I, OH or a reactively esterified OH group or another readily nucleophilically substitutable leaving group.

**11.** Process for the preparation of compounds of the formula I according to one or more of Claims 1 to 9, **characterised in that** a compound of the formula IV

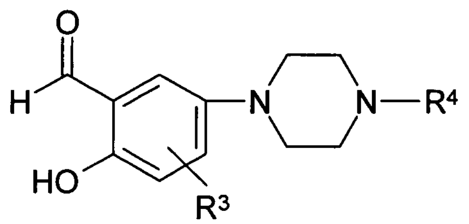

IV

in which $R^3$ has the meaning indicated in Claim 1, and $R^4$ stands for an amino-protecting group or H, is reacted, in a Michael-analogous reaction, with ethyl nitroacetate and diethylammonium chloride, and the nitro group is subsequently reduced, to give the compound of the formula V

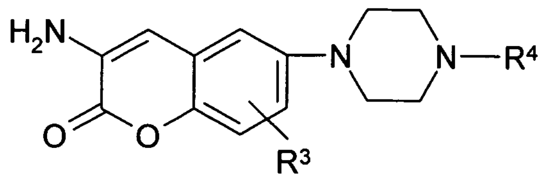

V

and the compound of the formula V is reacted with a compound conforming to the formula III.

**12.** Compounds of the formula I according to one or more of Claims 1 to 7 as medicaments.

**13.** Medicaments comprising an effective amount of a compound of the formula I according to one or more of Claims 1 to 9, besides optionally one or more inert excipients, adjuvants and/or diluents.

**14.** Medicaments according to Claim 13, **characterised in that** at least one further medicament active compound is present.

**15.** Process for the preparation of a medicament according to Claim 13 or 14, **characterised in that** a compound of the formula I according to one or more of Claims 1 to 9 and, if desired, a further medicament active compound is incorporated into one or more inert excipients and/or diluents by non-chemical methods.

**16.** Use of the compounds of the formula I according to one or more of Claims 1 to 9 for the preparation of a medicament for the prophylaxis and/or therapy of diseases selected from the group comprising depression, stroke, cerebral ischaemia, extrapyramidal motor side effects of neuroleptics and of Parkinson's disease, Alzheimer's disease, amyotrophic lateral sclerosis, brain and spinal cord trauma, obsessive-compulsive disorder, sleeping disorders, tardive dyskinesia, learning disorders, age-related memory disorders, eating disorders, such as bulimia, and/or sexual dysfunctions.

**17.** Medicament kit consisting of separate packs of

a) an effective amount of a compound of the formula I according to one or more of Claims 1 to 9 and
b) an effective amount of a further medicament active compound.

**Revendications**

**1.** Composés de formule I

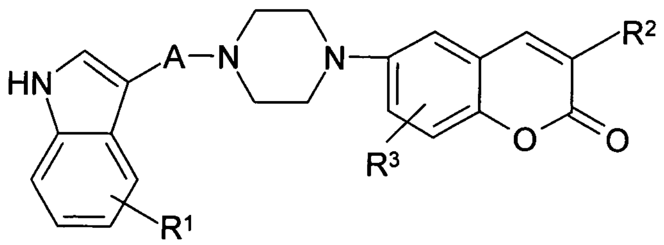

I

dans laquelle

$R^1$ désigne H, OH, CN, Hal, CONHR, OB, $CO_2B$, $CF_3$, $NO_2$, $NR_2$, NRCOR, NRCOOR, $NRCONR_2$,
$R^2$ désigne $NR_2$, NRCOR, NRCOOR, $NRCONR_2$, $NO_2$, $NRSO_2R_2$, NRCSR, $NRCSNR_2$,
$R^3$ désigne H, OH, CN, Hal, CONHR, OB, $CO_2B$, $CF_3$, $NO_2$, $NR_2$, NRCOR, NRCOOR, $NRCONR_2$,
R, indépendamment l'un de l'autre, désigne H, B, Hét, Ar,
Hét désigne un radical hétérocyclique mono-, bi- ou tricyclique, saturé, insaturé ou aromatique, non substitué ou mono- ou polysubstitué, où les hétéroatomes peuvent être S, N ou O et peuvent être présents de une à trois fois, ayant des substituants $NO_2$-, F-, Cl-, Br-, I-, HO-, $H_2N$-, $R^3HN$-, $(R^3)_2N$-, alkyl-, alkyl-O-, $CF_3$-O-, alkyl-CO-, aryl-, aryl-O-, aryl-CO-, aryl-CONH-, aryl- $SO_2$-, aryl-$SO_2$-HN-, où les substituants peuvent être présents, indépendamment l'un de l'autre, de 0 à 5 fois,
Ar désigne un radical hydrocarboné mono-, bi- ou tricyclique aromatique non substitué ou mono- ou polysubstitué, où des substituants convenables sont $NO_2$-, F-, Cl-, Br-, I-, HO-, $H_2N$-, $R^3HN$-, $(R^3)_2N$-, alkyl-, alkyl-O-, $CF_3$-O-, alkyl-CO-, aryl-, aryl-O-, aryl-CO-, aryl-CONH-, aryl$SO_2$-, aryl$SO_2$-HN- et les substituants peuvent être présents, indépendamment l'un de l'autre, de 0 à 5 fois,
A désigne une chaîne carbonée mono- ou polyinsaturée à chaîne linéaire ou ramifiée, ayant 2, 3, 4, 5 ou 6 atomes de C,
B désigne un radical alkyle à chaîne linéaire ou ramifiée ayant 1, 2, 3, 4, 5 ou 6 atomes de C,

et les solvats, stéréoisomères et sels pharmaceutiquement utilisables de ceux-ci, et des mélanges de ceux-ci en toutes proportions.

**2.** Composés de formule I selon la revendication 1, **caractérisés en ce que** le radical $R^1$ représente CN ou Hal, préférablement CN.

**3.** Composés de formule I selon la revendication 1 et/ou 2, **caractérisés en ce que** le radical $R^3$ représente H.

**4.** Composés de formule I selon l'une ou plusieurs des revendications 1 à 3, **caractérisés en ce que** le radical $R^2$ représente NRCOR ou NRCOOR.

**5.** Composés de formule I selon l'une ou plusieurs des revendications 1 à 4, **caractérisés en ce que** A représente $(CH_2)_m$, où m = 2, 3, 4, 5 ou 6, préférablement 4.

**6.** Composés de formule I selon l'une ou plusieurs des revendications 1 à 5, **caractérisés en ce que** le radical $R^1$ représente CN ou Hal, où CN est préféré, et $R^3$ représente H.

**7.** Composés de formule I selon l'une ou plusieurs des revendications 1 à 6, **caractérisés en ce que** $R^1$ représente

CN, $R^3$ représente H et A représente $(CH_2)_m$, où m = 4.

8. Composés de formule I selon l'une ou plusieurs des revendications 1 à 7, **caractérisés en ce que** le radical $R^1$ est en position 5 du radical indole.

9. Composés de formule I, choisis parmi le groupe constitué par le N-(6-{4-[4-(5-cyano-1H-indol-3-yl)butyl]pipérazin-1-yl}-2-oxo-2H-chromén-3-yl)méthylamide,
le (6-{4-[4-(5-cyano-1H-indol-3-yl)butyl]pipérazin-1-yl}-2-oxo-2H-chromén-3-yl)carbamate d'éthyle,
le N-(6-{4-[4-(5-cyano-1H-indol-3-yl)butyl]pipérazin-1-yl}-2-oxo-2H-chromén-3-yl)carbamate de méthyle,
le N-(6-{4-[4-(5-cyano-1H-indol-3-yl)butyl]pipérazin-1-yl}-2-oxo-2H-chromén-3-yl)-2,2-diméthylpropionamide,
le 3-{4-[4-(3-amino-2-oxo-2H-chromén-6-yl)pipérazin-1-yl]butyl}-1H-indole-5-carbonitrile,
et les pro-drogues, dérivés, solvats, stéréoisomères et sels pharmaceutiquement utilisables de ceux-ci.

10. Procédé de préparation de composés de formule I selon l'une ou plusieurs des revendications 1 à 9, **caractérisé en ce qu'**un composé de formule II

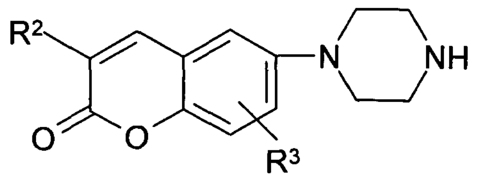

II

dans laquelle $R^2$ et $R^3$ ont les significations indiquées selon la revendication 1, est réagi avec un composé de formule III

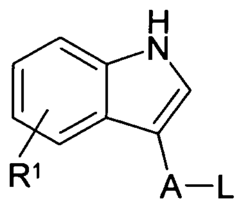

III

dans laquelle $R^1$ et A ont les significations indiquées selon la revendication 1, et L représente Cl, Br, I, OH ou un groupement OH estérifié de manière réactive ou un autre groupement partant pouvant être facilement substitué de manière nucléophile.

11. Procédé de préparation de composés de formule I selon l'une ou plusieurs des revendications 1 à 9, **caractérisé en ce qu'**un composé de formule IV

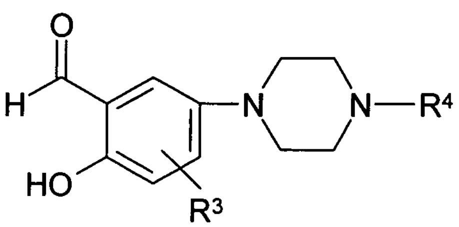

IV

dans laquelle $R^3$ a la signification indiquée selon la revendication 1, et $R^4$ représente un groupement protecteur d'amino ou H, est réagi, dans une réaction de type Michael, avec du nitroacétate d'éthyle et du chlorure de diéthylammonium, et le groupement nitro est ensuite réduit, pour donner le composé de formule V

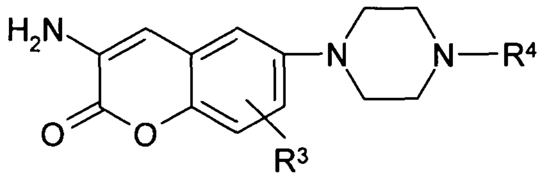

V

et le composé de formule V est réagi avec un composé répondant à la formule III.

**12.** Composés de formule I selon l'une ou plusieurs des revendications 1 à 7 comme médicaments.

**13.** Médicaments comprenant une quantité efficace d'un composé de formule I selon l'une ou plusieurs des revendications 1 à 9, outre éventuellement un ou plusieurs excipients, adjuvants et/ou diluants inertes.

**14.** Médicaments selon la revendication 13, **caractérisés en ce qu'**au moins un autre composé actif médicamenteux est présent.

**15.** Procédé de préparation d'un médicament selon la revendication 13 ou 14, **caractérisé en ce qu'**un composé de formule I selon l'une ou plusieurs des revendications 1 à 9 et, si on le souhaite, un autre composé actif médicamenteux, est incorporé dans un ou plusieurs excipients et/ou diluants inertes par des méthodes non chimiques.

**16.** Utilisation des composés de formule I selon l'une ou plusieurs des revendications 1 à 9 pour la préparation d'un médicament destiné à la prophylaxie et/ou la thérapie de maladies choisies parmi le groupe constitué par la dépression, les accidents vasculaires cérébraux, l'ischémie cérébrale, les effets secondaires sur le système moteur extrapyramidal induits par des neuroleptiques et par la maladie de Parkinson, la maladie d'Alzheimer, la sclérose latérale amyotrophique, les traumatismes du cerveau et de la moelle épinière, les troubles obsessionnels-compulsifs, les troubles du sommeil, la dyskinésie tardive, les troubles d'apprentissage, les troubles de mémoire liés à l'âge, les troubles de l'alimentation, tels que la boulimie, et/ou les dysfonctionnements sexuels.

**17.** Kit médicamenteux constitué de conditionnements séparés

a) d'une quantité efficace d'un composé de formule I selon l'une ou plusieurs des revendications 1 à 9 et
b) d'une quantité efficace d'un autre composé actif médicamenteux.

Fig. 1

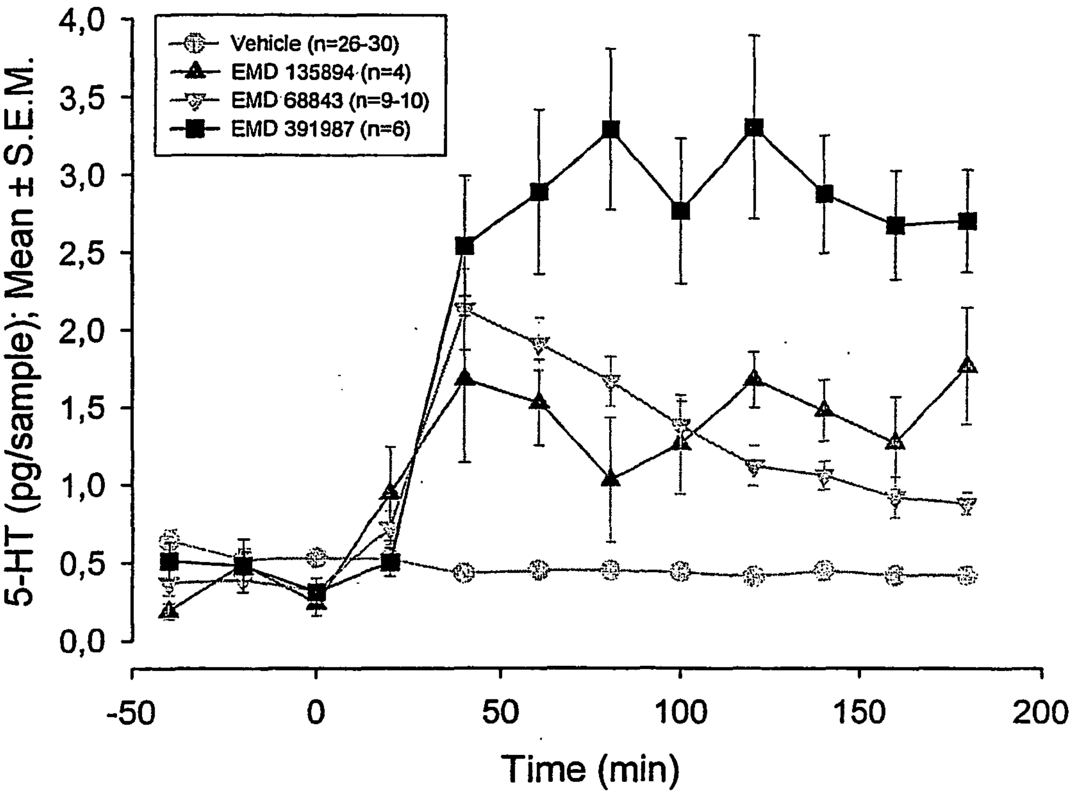
4,0
3,5
3,0
2,5
2,0
1,5
1,0
0,5
0,0
5-HT (pg/sample); Mean ± S.E.M.
Vehicle (n=26-30)
EMD 135894 (n=4)
EMD 68843 (n=9-10)
EMD 391987 (n=6)
-50
0
50
100
150
200
Time (min)

Fig. 2

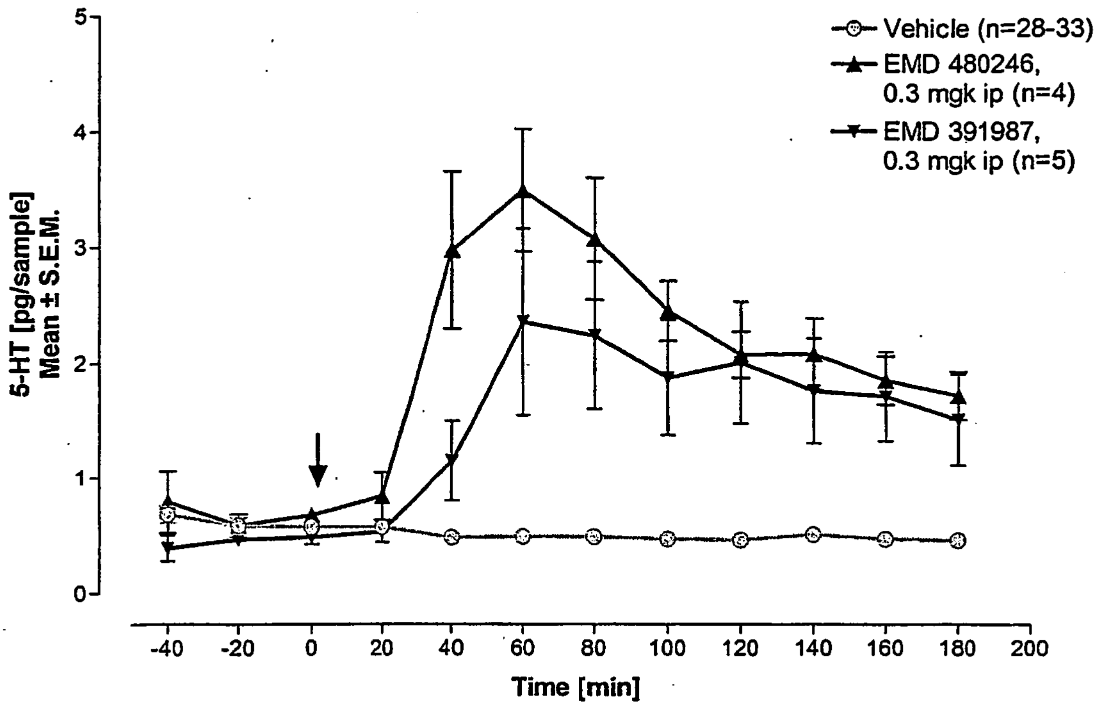

Vehicle (n=28-33)
EMD 480246, 0.3 mgk ip (n=4)
EMD 391987, 0.3 mgk ip (n=5)
5-HT [pg/sample] Mean ± S.E.M.
0
1
2
3
4
5
-40
-20
0
20
40
60
80
100
120
140
160
180
200
Time [min]

Fig. 3

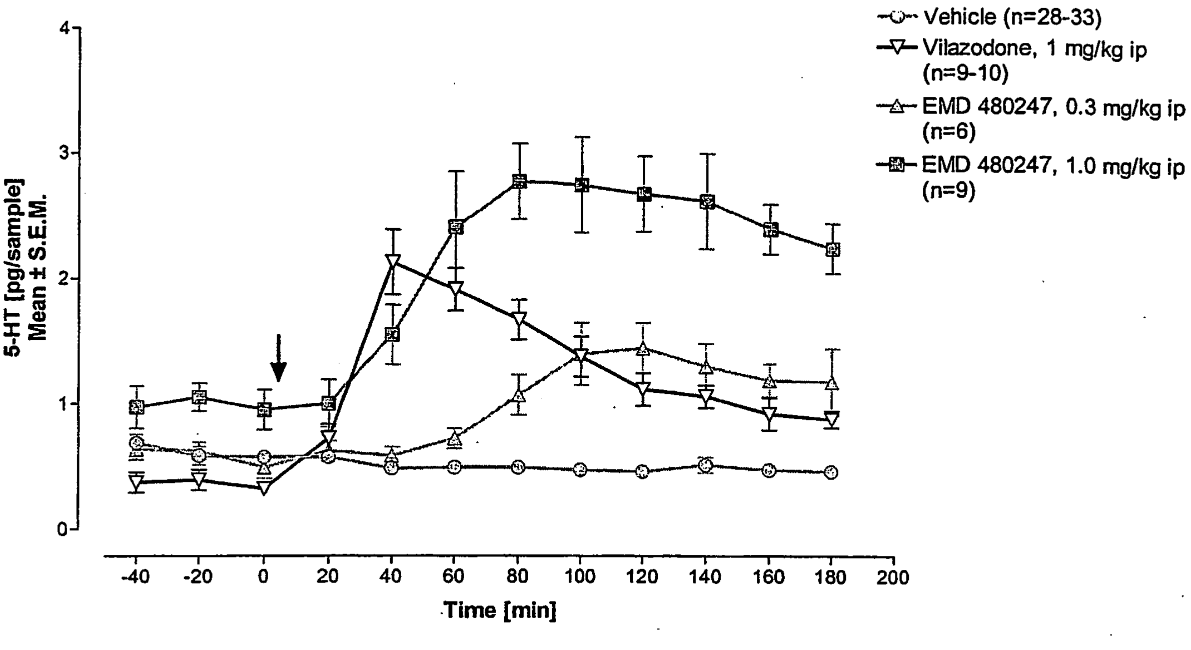
Vehicle (n=28-33)
Vilazodone, 1 mg/kg ip (n=9-10)
EMD 480247, 0.3 mg/kg ip (n=6)
EMD 480247, 1.0 mg/kg ip (n=9)
5-HT [pg/sample] Mean ± S.E.M.
0
1
2
3
4
-40
-20
0
20
40
60
80
100
120
140
160
180
200
Time [min]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19730989
- EP 0648767 B1


### In der Beschreibung aufgeführte Nicht-Patentliteratur


- **Cossery et al.** *European J. Pharmacol.,* 1987, vol. 140, 143-155
- **Sherman et al.** *Life Sci.,* 1978, vol. 23, 1863-1870
- **Seyfried et al.** *European J. Pharmacol.,* 1989, vol. 160, 31-41
- **Fozard ; Kilbinger.** *Br. J. Pharmacol.,* 1985, vol. 86, 601
- **DeVry.** *Psychpharmacol.,* 1995, vol. 121, 1-26
- **Wong et al.** *Neuropsychopharmacol.,* 1993, vol. 8, 23-33
- **Fulleret.** *J. Pharmacol. Exp. Ther.,* 1980, vol. 212, 115-119
- **Houben-Weyl.** Methoden der Organischen Chemie. Georg Thieme Verlag
- Organic Reactions. John Wiley & Sons, Inc,
- **Böttcher et al.** *J. Med. Chem.,* 1992, vol. 35, 4020-4026
- **Iyer et al.** *J. Chem. Soc. Perkin Trans. II,* 1973, 872-878
- *Angew. Chem.,* 1980, vol. 92, 129
- *Synthesis,* 1985, 184
- *J. Org. Chem.,* 1961, vol. 26, 1003
- **Di Chiara.** *Trends Pharmacol. Sci.,* 1990, vol. 11, 117-121